# EUROPÄISCHE PATENTSCHRIFT

(11) **EP 2 663 860 B1**
(45) Veröffentlichungstag und Bekanntmachung des Hinweises auf die Patenterteilung: **16.12.2015**
(21) Anmeldenummer: 12701056.9
(22) Anmeldetag: 10.01.2012
(51) Int. Cl.: G01N 33/487

(54) **MIKROSTRUKTURVORRICHTUNG ZUR MESSUNG AN MOLEKULAREN MEMBRANEN UND EIN VERFAHREN ZUR HERSTELLUNG DIESER MIKROSTRUKTURVORRICHTUNG**
MICROSTRUCTURE DEVICE FOR MEASURING MOLECULAR MEMBRANES AND A METHOD FOR PRODUCING SAID MICROSTRUCTURE DEVICE
DISPOSITIF MICROSTRUCTURÉ POUR MESURE SUR DES MEMBRANES MOLÉCULAIRES ET PROCÉDÉ DE FABRICATION DE CE DISPOSITIF MICROSTRUCTURÉ

(30) Priorität: 10.01.2011 DE 102011008206
(43) Veröffentlichungstag der Anmeldung: 20.11.2013
(73) Patentinhaber: Albert-Ludwigs-Universität Freiburg, 79095 Freiburg (DE)
(72) Erfinder: BEHRENDS, Jan, 79252 Stegen (DE); BAAKEN, Gerhard, 79102 Freiburg (DE); RÜHE, Jürgen, 79356 Eichstetten (DE); VELLINGER, Martin, 81925 München (DE)
(74) Vertreter: Kirchner, Christian
(86) Internationale Anmeldenummer: PCT/EP2012/000080
(87) Internationale Veröffentlichungsnummer: WO 2012/095298

(56) Entgegenhaltungen:
- EP-A1- 1 225 216
- WO-A1-2006/012571
- US-A1- 2002 144 905
- SIMON D. OGIER ET AL: "Suspended Planar Phospholipid Bilayers on Micromachined Supports", LANGMUIR, Bd. 16, Nr. 13, 1. Juni 2000 (2000-06-01), Seiten 5696-5701, XP55023997, ISSN: 0743-7463, DOI: 10.1021/la991367o
- BAAKEN G ET AL: "Planar microelectrode-cavity array for high-resolution and parallel electrical recording of membrane ionic currents", LAB ON A CHIP, ROYAL SOCIETY OF CHEMISTRY, CAMBRIDGE, GB, Bd. 8, Nr. 6, 1. Juni 2008 (2008-06-01), Seiten 938-944, XP007919564, ISSN: 1473-0197, DOI: 10.1039/B800431E [gefunden am 2008-06-01] in der Anmeldung erwähnt
- GERHARD BAAKEN ET AL: "Nanopore-Based Single-Molecule Mass Spectrometry on a Lipid Membrane Microarray", ACS NANO, Bd. 5, Nr. 10, 25. Oktober 2011 (2011-10-25), Seiten 8080-8088, XP55023858, ISSN: 1936-0851, DOI: 10.1021/nn202670z

## Beschreibung

Die vorliegende Erfindung betrifft eine Mikrostrukturvorrichtung zur Messung an molekularen Membranen, insbesondere Bilipidschichten, und ein Verfahren zur Herstellung dieser Mikrostrukturvorrichtung.

Molekulare Membranen, insbesondere Bilipidschichten (Doppel-Lipidschichten), werden z.B. in der Membranbiophysik und zellulären Elektrophysiologie ebenso wie für bestimmte einzelmolekülanalytische Verfahren, die auf Nanoporen beruhen (molekularer Coulter-Counter), verwendet. Bei diesen Anwendungen setzt man insbesondere die Spannungsklemmtechnik ("voltage-clamp") ein, um eine präzise Messung von Flüssen geladener Teilchen (Ionen) zu erzielen. Die Messung des Ionenstroms erfolgt über eine dielektrische (isolierende) Trennschicht (Membran) zwischen zwei Elektrolytlösung enthaltenden Kompartimenten, die mindestens eine für Ionen durchlässige Pore bzw. einen Ionenkanal enthält. Diese Membran kann eine Bilipidschicht sein, die der typische Grundbestandteil natürlicher biologischer (Zell-)Membranen ist und die deshalb als künstliches Modell einer natürlichen Zellmembran herangezogen werden kann. Für die Spannungsklemmtechnik ist es erforderlich, zwei Kompartimente, nämlich die elektrolytgefüllten Bereiche diesseits und jenseits der Membran, elektrisch zu kontaktieren. Zu diesem Zweck wird die Membran meist über der Mikroapertur einer Trägersubstratoberfläche erzeugt, wobei die Mikroapertur den oberen Rand einer Mikrokavität oder eines Mikrolochs in diesem Trägersubstrat bildet. Bringt man die Bilipidschicht über dieser Oberfläche auf, überspannt diese Molekülschicht die Mikroapertur "freitragend" mittels der ihr eigenen Oberflächenspannung. Da diese Mikroaperturen im optischen Mikroskop "schwanz" erscheinen, werden sie auch "Black Lipid Membranes" (BLM) genannt.

Sowohl für eine hohe Präzision der Messung (hohes Signal- zu Rausch-Verhältnis) sowie für einen hohen Durchsatz von Voltage-Clamp Messungen ist es wünschenswert, solche Messungen in Mikrostrukturen durchzuführen, in denen die dielektrische Trennschicht integriert ist. Je kleiner die Abmessungen der Trennschicht sowie der elektrolytgefüllten Kompartimente, desto geringer ist das elektrische Messrauschen und desto mehr solcher Messanordnungen lassen sich in Form eines Arrays auf kleiner Fläche unterbringen. Eine hohe Dichte solcher Anordnungen ist Voraussetzung für Messungen mit hohem Durchsatz.

Alle etablierten Vorrichtungen für Voltage-Clamp Messungen an dielektrischen Trennschichten bzw. (Zell)Membranen beruhen auf dem strukturellen Prinzip, dass die Membran (Zellmembran, synthetische Lipidmembran, SiO2-Schicht, SiN3-Schicht, Graphenschicht o.ä.) eine Mikroapertur elektrisch möglichst dicht verschließt, die in einer elektrisch isolierenden Trägerschicht ausgebildet ist, die zwei mit elektrisch leitfähigem Medium (Salzlösung, Elektrolyt) gefüllte Kompartimente voneinander trennt. Die elektrische Kontaktierung, also der Übergang von elektronischer zu ionischer Leitung erfolgt jeweils an einer von zwei Redox-Elektroden (typischerweise Ag/AgCl) die jeweils in eines der beiden Kompartimente hineinreichen oder über Salzbrücken damit verbunden sind. Typischerweise handelt es sich dabei um mit Silberchlorid beschichtete Silberdrähte mit Dicken zwischen 0,5 und 2 mm. Um diese in elektrischen Kontakt mit der Membran zu bringen sind relativ große, elektrolytgefüllte Räume notwendig, die Platz benötigen und damit die Integrationsdichte limitieren. Zusätzlich bedingt eine große von Elektrolyt benetzte Fläche eine hohe elektrische Kapazität des Gesamtsystems, die sich über das kapazitive Stromrauschen negativ auf die Messgenauigkeit auswirkt (parasitäre Kapazität).

Eine stark vereinfachte Mikrostruktur für Spannungsklemmtechnik-Messungen an Membranen wurde deshalb jeweils vorgeschlagen in Baaken, Prucker, Behrends, Rühe 2005, European Cells and Materials 5, Suppl. 5, p. CS4; Baaken, Prucker, Sondermann, Behrends, Rühe 2007, Tissue Engineering 18, 889; bzw. im Dokument "Baaken et al." (Baaken et al., "Planar microelectrode-cavity array for high-resolution and parallel electrical recording of membrane ionic currents", Lab Chip, 2008, 8, 938-944). Bei der dort beschriebenen Mikrostruktur wurde im Gegensatz zu früheren Verfahren die Membran nicht auf einer Mikroapertur zwischen zwei Kompartimenten, sondern auf der Öffnung einer in einem elektrisch isolierenden Material eingebrachten Kavität (in Form eines Sackloches) aufgebracht. Die elektrische Kontaktierung des einige pL bis einige 100 fL großen Elektrolytvolumens innerhalb der Kavität erfolgt dabei insbesondere mit Hilfe einer am Boden derselben mikrogalvanisch ausgebildeten Ag/AgCI-Mikroelektrode (Mikroelektroden-Kavitäten-Arrays, MECA). Eine ähnliche Mikrostrukturvorrichtung beschreibt auch die US 2009/0167288 A1.

Durch diese Vereinfachung sinkt der Platzbedarf pro Messposition, was sehr viel höhere Integrationsdichten als bisher ermöglicht und zudem die elektrischen Parameter (Kapazität, Zugangswiderstand) optimiert; zusätzlich werden die Herstellungskosten gesenkt.

Bisher ist dieser vielversprechende Ansatz allerdings mit einem grundsätzlichen Problem behaftet, das seine praktische Eignung für Messungen im Hochdurchsatz und seine Zuverlässigkeit stark in Frage stellt: insbesondere für die Messung größerer Ströme wie sie bei Messungen an ganzen Zellen (kurzzeitig > 1 nA) sowie bei Messungen an Nanoporen (dauerhaft > 100 pA) auftreten, sind leine Ag/AgCl Mikroelektroden, insbesondere mit Durchmessern von < 50 µm, nicht zufriedenstellend stabil. Dies beruht auf der Tatsache, dass bei kleinen Elektrodenflächen sehr hohe Stromdichten mit entsprechender Intensität des chemischen Massenumsatzes an der Elektrode auftreten Dadurch wird bereits nach wenigen Minuten aufgrund der Redox-Reaktionen je nach Polarität der Elektrode entweder eine zunehmend dicke, schlecht leitende AgCI-Schicht aufgebaut (bei Schaltung als Anode) oder das AgCl so weit abgebaut, daß nur noch reduziertes Ag vorliegt (bei Schaltung als Kathode). Im ersten Fall steigt der Widerstand der Elektrode massiv an, im zweiten ist nurmehr kapazitive Kopplung an das Elektrolyt nach Art einer polarisierbaren Elektrode möglich und der DC-Widerstand steigt abrupt. Wünschenswert wäre ein möglichst konstantes, driftarmes elektrisches Verhalten der Elektrode.

Die Publikation S.D. Ogier et al "Suspended Planar Phospholipid Bilayers on Micromachined Supports", Langmuir, Bd. 16, Nr. 13, 1.Juni 2000, S. 5696-5701 beschreibt eine Vorrichtung wie im Oberbegriff des Anspruchs 1 genannt.

Es ist Aufgabe der vorliegenden Erfindung, eine verbesserte Mikrostrukturvorrichtung und ein Verfahren zu deren Herstellung bereitzustellen, deren elektrische Messeigenschaften im Elektrolyt bei Messung an einer molekularen Membran insbesondere länger möglichst stabil sind.

Diese Aufgabe wird erfindungsgemäß gelöst durch die Mikrostrukturvorrichtung gemäß Anspruch 1 und das Verfahren gemäß Anspruch 7. Bevorzugte Ausgestaltungen des Verfahrens bzw. der in diesem verwendeten Vorrichtung sind Gegenstände der Unteransprüche 2 bis 6 und 8.

Verwendet man die erfindungsgemäße Mikrostrukturvorrichtung z.B. zur Messung an einer molekularen Membran mittels Spannungsklemmtechnik, wie eingangs beschrieben, ergibt es sich, dass der durch Elektrode und Membran fließende Strom an der Elektrode eine relativ geringe Stromdichte bewirkt, da deren Kontaktseite mit dem Elektrolyt einen relativ großen charakteristischen Durchmesser aufweist. Die relativ geringe elektrische Stromdichte (Strom/Fläche) der Kontaktseite bringt den Vorteil mit sich, dass die pro Fläche elektrochemisch induzierten Reaktionen an der Kontaktseite der Elektrode reduziert werden, welche die Elektrode mit der Zeit umbilden und so deren Eigenschaften ändern. Auf diese Weise kann insbesondere bei Verwendung einer Silber/Silberchlorid-Elektrode die Bildung der nachteilig dicken Silberchloridschicht (Anodenschaltung der Elektrode) oder die nachteilige totale Rückbildung der Silberchloridschicht (Kathodenschaltung der Elektrode) verzögert werden. Dadurch weist die Elektrode konstantere elektrische Eigenschaften auf, insbesondere eine geringere Drift und eine verlängerte Messdauer (bevor die Elektrode gegebenenfalls durch Umpolung zumindest teilweise wieder restauriert werden kann).

Als charakteristischer Durchmesser einer Seite oder Fläche wird vorliegend der Durchmesser einer Kreisfläche verstanden, welche dieselbe absolute Größe hat wie diese Seite oder Fläche. Z. B. ist der charakteristische Durchmesser D eines Quadrats mit der Seitenlänge a ist D=2*a/sqrt(pi) und der charakteristische Durchmesser D eines Kreises mit dem Radius D/2 ist D.

Die Richtungsbezeichnung "oben" bzw. "nach oben" bezeichnet vorliegend die Richtung des Normalenvektors, der aus der Ebene der Mikroapertur von der Mikrokavität wegweist. Diese Richtung wird auch als die Richtung der positiven z-Achse eines kartesischen Koordinatensystems definiert (der Ursprung dieses Koordinatensystems wird hierdurch vorzugsweise nicht festgelegt). Die Richtungsbezeichnung "unten" bzw. "nach unten" bezeichnet vorliegend die Richtung des Normalenvektors, der aus der Ebene der Mikroapertur in die Mikrokavität hinein weist (auch bezeichnet als die Richtung der negativen z-Achse dieses Koordinatensystems). Die Ebene der Mikroapertur ist die Ebene, die mit der von einer planaren Mikroapertur umrahmten Fläche (Mikroaperturfläche) coplanar liegt. Die Mikroapertur liegt vorzugsweise im wesentlichen vollständig in einer Ebene, die insbesondere parallel zur x-y-Ebene dieses Koordinatensystems verläuft. Die Mikroapertur kann aber auch in mehr als einer Ebene verlaufen, insbesondere einen kontinuierlichen (stufenfreien) Verlauf aufweisen.

Der charakteristische Durchmesser D2 der Kontaktseite der Elektrode ist vorzugsweise größer als D1*c (D1 multipliziert mit einem Faktor c), wobei c jeweils vorzugsweise beträgt: 1,1; 1,2; 1,3; 1,4; 1,5; 2,0; 3; 4; 5; 10; 15; 20; 25, 35 oder 50. Das Verhältnis D2/D1 kann aber auch größer sein. Bereits bei einem Verhältnis von D2/D1=1,1 konnte eine Verbesserung der Stabilität der Elektrode gegenüber dem Fall D2/D1=1,0 beobachtet werden. Bevorzugt ist aber insbesondere, wenn D2 mindestens 1,5 mal größer ist als D1 (D2>1,5*D1) und -vorzugsweise- kleiner ist als 20 (D2<20). Es ergeben sich dann besonders geeignete Elektrodeneigenschaften bei -mit photolithographischen Mitteln- leicht herzustellenden Mikrostrukturen. Die stabilsten Elektroden ergaben sich bei 2,0 < D2/D1 < 20. Wird D1 konstant gehalten, so muss bei Verhältnissen D2/D1>20 in Kauf genommen werden, dass der Flächenbedarf für die einzelne Elektrode und Messstelle relativ groß wird, so dass die mögliche Dichte der Elektroden pro Trägersubstratfläche abnimmt. Dennoch können auch solche großen Verhältnisse D2/D1 vorteilhaft sein, insbesondere hinsichtlich der Stabilität der Elektrodeneigenschaften.

D2/D1=1 könnte zwar grundsätzlich auch verwendet werden. Es würde aber z.B. eine einfache Vergrößerung des Durchmessers der Mikrokavität und damit sowohl der den Boden derselben bildenden Elektrode wie auch der Mikroapertur zwar die Stromdichten verringern und die Elektrodenstabilität verbessern, würde aber das Problem nicht zufriedenstellend lösen, da insbesondere für hochauflösende Messungen an Nanoporen kleine Mikroaperturdurchmesser zu bevorzugen sind (insbesondere < 50 µm) und kleine Mikroaperturdurchmesser (z.B. < 10 µm) insbesondere für Messungen an Zellen zu bevorzugen sind.

Vorzugsweise weist das Trägersubstrat eine erste Schicht auf, innerhalb der die Elektrode zumindest teilweise oder im wesentlichen vollständig angeordnet ist. Eine Schicht kann eine Schicht im Trägersubstrat sein oder eine Beschichtung des Trägersubstrats sein. Eine solche Schicht ist vorzugsweise zumindest teilweise oder vollständig im wesentlichen planar. Falls die Elektrode vollständig in der ersten Schicht angeordnet ist, bedeutet dies vorzugsweise, dass kein Abschnitt der Elektrode aus der ersten Schicht hervorsteht bzw. nicht die gedachten Hauptebenen durchdringt, die die Schicht z.B. nach oben und nach unten einhüllen. Das Trägersubstrat kann mindestens eine Schicht aufweisen, insbesondere mehr als zwei Schichten oder eine Vielzahl von Schichten, wobei die Mikrokavität in einer dieser Schichten, mehrerer dieser Schichten oder jeder dieser Schichten angeordnet sein kann oder nicht angeordnet sein kann.

Die erste und/oder zweite Schicht des Trägersubstrats ist vorzugsweise eine Beschichtung des Trägersubstrats, die vorzugsweise dessen Oberseite bildet und vorzugsweise hydrophob ist, besteht vorzugsweise aus einer lichtempfindlichen Schicht, insbesondere ein Epoxidharz oder Fotolack, oder weist diese auf. Die Beschichtung besteht vorzugsweise aus einem Polymer, vorzugsweise aus Epoxidharz oder vorzugsweise aus einem Fotolack, z.B. SU8 oder weist ein solches Material auf. Dieser Fotolack ist vorzugsweise SU8, dessen getrocknete Schichten hydrophob sind. SU-8 ist ein kommerziell erhältlicher Fotolack der Firma Microchem Corp., USA, und gehört zu der Gruppe der Negativ-Resiste. Wie die meisten Resiste besteht SU-8 aus den drei Bestandteilen Grundharz, Lösungsmittel und fotoempfindlicher Komponente. Diese eignen sich besonders zur Herstellung der Mikrostrukturvorrichtung, da sich insbesondere viele Bilipidschichten auf diesen Resisten besonders zuverlässig ausbilden und da diese photolitographisch bearbeitbar sind, um z.B. Mikrostrukturen herzustellen, und chemisch relativ inert sind.

Die Beschichtung des Trägersubstrats kann ferner vorzugsweise Polytetrafluorethylen (PTFE) aufweisen oder aus diesem Material bestehen. Der Vorteil ist, dass solche Schichten chemisch besonders inert sind, wodurch sie sich besonders zur Anwendung in korrosiven Umgebungen eignen (z.B. physiologischen Elektrolyten- physiologischen Salzlösungen).

Vorzugsweise weist die Mikrostrukturvorrichtung mindestens eine Zuleitungseinrichtung auf, mittels der die Elektrode über eine Zuleitungsdistanz elektrisch kontaktierbar ist. Vorzugsweise ist einer (oder jeder) Elektrode mindestens eine Zuleitungseinrichtung zugeordnet. Eine Zuleitungseinrichtung kann eine metallische Leiterbahn aufweisen oder aus dieser bestehen, die z.B. aus Gold, Titan, Nickel-Chrom, Platin, oder Silber bestehen kann oder eines oder mehrere dieser Materialien aufweist. Zur Unterscheidung von Elektrode und Zuleitungseinrichtung kann definiert werden, dass die mit dem Elektrolyt oder die mit dem Innenvolumen der Mikrokavität in Kontakt stehenden Teile des Systems aus Elektrode und Zuleitungseinrichtung der Elektrode zugerechnet werden während die nicht mit dem Elektrolyt oder mit dem Innenvolumen der Mikrokavität in Kontakt stehenden Teile des Systems aus Elektrode und Zuleitungseinrichtung der Zuleitungseinrichtung zugerechnet werden. Vorzugsweise ist die Zuleitungseinrichtung vollständig oder zumindest zum Teil in der ersten Schicht angeordnet.

Die Elektrode bildet vorzugsweise zumindest einen Teil der Innenwandung der Mikrokavität. Die Mikrokavität ist vorzugsweise derart ausgebildet, dass das Innenvolumen der Mikrokavität von der durch die Mikroapertur umrahmten Fläche und der Innenwand (oder mehrerer Innenwände) der Mikrokavität begrenzt wird. Die mindestens eine Innenwand der Mikroapertur wird vorzugsweise aus seitlichen Innenwänden und einer Bodenwand gebildet. Die Bodenwand der Mikrokavität wird vorzugsweise vollständig oder zumindest teilweise von der Kontaktseite der Elektrode gebildet. Die Elektrode kann aber auch zumindest teilweise oder vollständig vom Innenvolumen der Mikrokavität umgeben sein. Z.B. kann die Elektrode teilweise freitragend angeordnet sein.

Vorzugsweise ist diese erste Schicht im wesentlichen unterhalb oder zumindest teilweise unterhalb der Mikrokavität angeordnet. Dadurch wird insbesondere die einfache Herstellung der Elektrode ermöglicht, welche vorzugsweise unterhalb der Mikrokavität angeordnet ist.

Vorzugsweise weist das Trägersubstrat eine zweite Schicht auf, innerhalb der die Mikrokavität wenigstens teilweise oder im wesentlichen vollständig angeordnet ist. Dadurch wird insbesondere die einfache Herstellung der Mikrokavität ermöglicht, welche vorzugsweise oberhalb der Elektrode angeordnet ist. Es ist aber auch bevorzugt, dass die Mikrokavität zumindest teilweise, vorzugsweise mit weniger als der Hälfte oder einem Viertel ihres Innenvolumens, auch in der ersten Schicht angeordnet ist.

Es weist die Mikrokavität einen zweiten, oberen, Raumabschnitt auf, der von der Mikroaperturfläche begrenzt ist und der (zumindest in einer Höhe oder im Durchschnitt aller Höhen) im Querschnitt einen charakteristischen Durchmesser D3 aufweist. Ferner weist die Mikrokavität einen ersten, unteren, Raumabschnitt auf, der unterhalb des oberen Raumabschnitts liegt und der (zumindest in einer Höhe oder im Durchschnitt aller Höhen) in diesem Querschnitt einen charakteristischen Durchmesser D4 aufweist, wobei D4 größer ist als D3 (D4>D3), nämlich insbesondere D4 größer ist als D3*c (D3 multipliziert mit einem Faktor c), wobei c jeweils vorzugsweise beträgt: 1,5; 2,0; 3; 4; 5; 10; 15; 20; 25; 50, und ferner vorzugsweise im wesentlichen D4=D2 und im wesentlichen D3=D1 ist (der betrachtete Querschnitt führt durch den geometrischen Mittelpunkt einer planaren Mikroaperturfläche und liegt zu dieser senkrecht). Ein Raumabschnitt ist vorzugsweise im wesentlichen zylinderförmig, wobei die Zylinderachse vorzugsweise parallel zur z-Achse verläuft. Ein Raumabschnitt kann aber auch im wesentlichen quaderförmig sein oder anders ausgebildet sein.

Die charakteristische Höhe H3 des zweiten Raumabschnitts beträgt vorzugsweise zwischen 1 µm und 50 µm, vorzugsweise zwischen 1 µm und 10 µm oder zwischen 3 µm und 6 µm, kann aber auch anders bemessen sein. Die charakteristische Höhe H4 des ersten Raumabschnitts beträgt vorzugsweise zwischen 0 µm und 50 µm, vorzugsweise zwischen 0 µm und 10 µm, zwischen 3 µm und 6 µm, besonders bevorzugt zwischen 0,0005 µm und 1,0 µm oder 0,0005 µm und 0,5 µm oder zwischen 0,0005 µm und 0,05 µm oder zwischen 0,001 µm und 0,05 µm, kann aber auch anders sein. H4 ist kleiner als H3. Der untere Raumabschnitt dient vorzugsweise dem Kontaktieren der relativ großen Kontaktseite der Elektrode mit charakteristischen Durchmesser D2 durch den Elektrolyten. Dazu sollte möglichst die gesamte Kontaktseite der Elektrode kontaktierbar sein, indem vorzugsweise im wesentlichen D4=D2 ist, während die Höhe H4 des unteren Raumabschnitts für das elektrische Kontaktieren der Kontaktseite durch den Elektrolyten nicht groß sein muss. Entsprechend kann H4 klein gewählt werden. Dadurch ergeben sich bei kleinen Werten H4 geringere Abstände der Elektrode von der Mikroapertur, welche die zu messende Membran tragen soll. Dadurch wird insbesondere bei deren Verwendung für die Spannungs-Klemmtechnik ermöglicht, dass das Signal-Rausch-Verhältnis verbessert wird. Ferner kann die Mikrostrukturvorrichtung bei kleinen Werten H4 insbesondere kompakt gehalten werden. Vorzugsweise ist der erste bzw. untere Raumabschnitt vollständig innerhalb der ersten

Schicht angeordnet und vorzugsweise ist der zweite bzw. obere Raumabschnitt vollständig innerhalb der zweiten Schicht angeordnet. Dadurch lässt sich ein Raumabschnitt einfach fertigen, indem dieser durch fotolithographische Mittel (insbesondere durch die Verwendung einer Maske und UV-Licht) aus der Schicht entfernt werden, die vorzugsweise aus einem Fotolack (z.B. SU8) besteht.

Die "charakteristische Höhe" H4 (oder H3) des Raumabschnitts ist vorzugsweise ein Durchschnittswert. Er ergibt sich vorzugsweise daraus, dass dem Raumabschnitt ein fiktives (Zylinder-)Volumen V zugeordnet wird, das sich aus dem Produkt einer charakteristischen Kreisfläche A und dieser charakteristischen Höhe H4 ergibt (V=A*H4 bzw. V=A*H3). Davon abgesehen kann ein Raumabschnitt tatsächlich zylinderförmig sein. Die charakteristische Kreisfläche A ergibt sich dabei aus dem charakteristischen Durchmesser D4 (oder D3) (A=(D4/2)^2*pi). Der charakteristische Durchmesser des Raumabschnitts wird, insbesondere bei konstantem seitlichem Maß oder konstanten Durchmessern des Raumabschnitts, vorzugsweise zumindest in einer Höhe betrachtet oder, insbesondere bei variablen seitlichen Maßen oder bei in Abhängigkeit von dessen Höhe veränderlichen Durchmessern des Raumabschnitts, vorzugsweise als Durchschnitt der charakteristischen Durchmesser in allen Höhen dieses Raumabschnitts betrachtet. Zum Beispiel ist der charakteristische Durchmesser Dk eines rotationssymmetrischen Kegels mit einem Durchmesser D der Kreisgrundfläche und einer Höhe h Dk=D/4 und der charakteristische Durchmesser Dz eines Zylinders mit Durchmesser D der Kreisgrundfläche des Zylinders beträgt Dz=D.

Als Raumabschnitt wird insbesondere ein zusammenhängendes Innenvolumen der Mikrokavität angesehen, das insbesondere mit Wasser oder einem wässrigen Elektrolyten gefüllt werden kann. Es hat sich in Experimenten überraschend gezeigt, dass insbesondere geringe oder sehr geringe Höhen H4 (z.B. 0,0005 µm < H4 < 0,5 µm) ausreichend sind, um eine Elektrode mit relativ großem charakteristischen Durchmesser D2>D1 elektrisch zu kontaktieren. Dies wurde insbesondere durch solche Volumina des zweiten Raumabschnitts erreicht, die insbesondere durch ein offenporiges oder schwammartiges Füllmaterial definiert sind, indem das Volumen durch das zusammenhängende Innenvolumen der Poren gebildet ist. Der zweite Raumabschnitt kann durch ein offenporiges oder schwammartiges Füllmaterial definiert sein. Dieses Volumen des zweiten Raumabschnitts kann z.B. von der Elektrode und von Innenwandabschnitten, die durch das Trägersubstrat definiert sind und die zylinderförmig sein können, sowie von der Unterseite des ersten Raumabschnitts eingerahmt sein.

Vorzugsweise weist die Kontaktseite der Elektrode eine zumindest in einem Abschnitt (oder überall), und insbesondere auf mikroskopischer oder nanoskopischer Skala, nichtplanare Kontaktgrenzfläche zum Innenvolumen der Mikrokavität auf, wobei diese Kontaktgrenzfläche größer ist als die Fläche der makroskopisch planaren Kontaktseite in diesem Abschnitt. Dies kann durch Erhebungen (streifenförmig, fleckförmig, plateauartig) oder Vertiefungen (rinnenartig, lochartig, kanalartig) der Kontaktseite erreicht werden. Es kann ferner durch Mikrostrukturierung (charakteristische Abstände der Oberflächenstrukturen im Bereich von 1µm bis 1000 µm) oder Nanostrukturierung (charakteristische Abstände der Oberflächenstrukturen im Bereich von 0,5 nm bis 1000 nm) der Kontaktseite der Elektrode erreicht werden oder durch eine Materialeigenschaft der Kontaktseite (z.B. Rauheit, Porosität, insbesondere Mikroporosität oder Nanoporosität, wie bei Ag/AgCI-Elektrode). Durch Vergrößerung der Elektrodengrenzfläche kann im Betrieb der Elektrode die elektrische Stromdichte (elektrischer Strom/ Elektrodengrenzfläche) an der Elektrode reduziert werden und die Elektrode so stabiler gehalten werden, z.B. bei einer Spannungsklemmtechnik-Messeranordnung der Mikrostrukturvorrichtung.

Es hat sich ferner gezeigt, dass insbesondere elekrochemisch hergestellte Elektroden aus dem Material Silber/Silberchlorid (Ag/AgCl) als typische Eigenschaft eine nanoporöse Schicht aufweisen, die ein zusammenhängendes Innenvolumen bildet, das insbesondere von einem in der Mikrokavität angeordneten Elektrolyt gefüllt wird. Der nanoporösen Schicht lässt sich somit ein zweiter Raumabschnitt mit charakteristischer Höhe H4 und charakteristischem Durchmesser D4 zuordnen. Die nanoporöse Schicht besteht aus nanoporösem AgCl oder weist AgCl auf. Typische Korngrößen der nanoporösen Schicht betragen 50 nm bis 500 nm. Eine Ag/AgCI-Elektrode wird vorzugsweise durch elektrochemische Abscheidung aus einer Silbernitratlösung erzeugt. Weitere Details dazu finden sich z.B. im Dokument "Baaken et al.". Die Porosität ist ein Standard-Ergebnis der Mikrogalvanik, während der das Silber elektrochemisch auf eine z.B. Goldstartschicht abgeschieden wird. Die Porosität ist abhängig von den elektrischen Parametern (Stromstärke, Spannung), Dauer und Konzentration der verwendeten Elektrolyte (Silbernitratlösungen). Bei leichter Änderung z.B. höheren Konzentrationen der Silbernitratlösung werden die abgeschiedenen "Silberkristalle" deutlich größer.

Es ist zu beachten, dass sich das Volumen und die Höhe des zweiten Raumabschnitts im Betrieb der Mikrostrukturvorrichtung ändern werden, wenn die Mikrostrukturvorrichtung eine Elektrode aufweist, die sich im Elektrolyten in Abhängigkeit von der elektrischen Polung der Elektrode verändert. Dies ist bei Ag/AgCI-Elektroden der Fall, wie eingangs erläutert. Da aber auch das nanoporöse AgCl, welches auf der Elektrode in Abhängigkeit von deren Polung zunimmt oder abnimmt, ausreichend offenporig nanoporös ist, also ein ausreichend zusammenhängendes Innenvolumen bildet, bleibt im wesentlichen die gesamte Elektrodenfläche mit charakteristischen Durchmesser D2 selbst dann elektrisch kontaktiert, wenn ein ursprünglich z.B. hohlzylinderartiger zweiter Raumabschnitt später im wesentlichen vollständig von dem nanoporösen Material gefüllt ist.

Das Trägersubstrat ist vorzugsweise zumindest abschnittsweise oder im wesentlichen vollständig planar ausgebildet. Das Trägersubstrat weist eine oder mehrere Mikrostrukturen auf, also räumliche Hervorhebungen und/oder Vertiefungen mit kleinen Dimensionen, z.B. diese Mikrokavitäten die z.B. wenige Nanometer, einige wenige Mikrometer, einige wenige zehn Mikrometer oder einige wenige hundert Mikrometer betragen können. Solche Mikrostrukturen lassen sich z.B. durch bekannte optischlitographische Verfahren erzeugen, bei denen mittels optischer Masken definierte Strukturen schichtweise auf einem Trägersubstrat aufgebracht werden und teilweise wieder entfernt werden.

Das Trägersubstrat weist vorzugsweise eine Oberseite auf, die vorzugsweise zumindest abschnittsweise oder im wesentlichen vollständig planar ist. Die Oberseite weist vorzugsweise mindestens eine Mikroapertur auf, vorzugsweise eine Anzahl N von Mikroaperturen, wobei N jeweils vorzugsweise zwischen 2 und 2000, größer als 2000, vorzugsweise zwischen 2 und 400, zwischen 4 und 100, zwischen 4 und 50 oder zwischen 4 und 20 liegt. Über einer Mikroapertur kann eine "freitragende" molekulare Membran (BLM), z.B. eine Bilipidmembran hergestellt werden. Dies erfolgt bekanntermaßen z.B. durch "Aufstreichen" ("Streichmethode", "Painting") einer Lösung aus erstem Lösungsmittel (z.B. Hexan, Heptan, Oktan, Nonan, Dekan, Hexadekan oder andere Alkane, oder einer Mischung aus einem oder mehrerer dieser Stoffe) und diesem Lipid in einer Konzentration von z.B. 1 mg/ml auf dem Trägersubstrat über dieser Mikroapertur. Die freitragende Molekülschicht trennt dann zwei Kompartimente voneinander, was Messanordnungen zur Realisierung einer Spannungsklemmtechnik ermöglicht. Die Verwendung mehrere Mikroaperturen und Mikrokavitäten hat den Vorteil, dass mehrere solcher Sensoriken parallel betrieben werden können, was einen höheren Messdurchsatz erlaubt.

Eine solche molekulare Membran kann eine Schicht aus amphiphilen Molekülen, insbesondere Lipide, aufweisen oder daraus bestehen. Die Schicht kann insbesondere eine Doppelschicht sein, also aus zwei übereinander angeordneten Einzelschichten bestehen, wobei eine Einzelschicht insbesondere aus selbstorganisierten Molekülen besteht. Eine solche molekulare Membran kann künstlich hergestellt sein, insbesondere kann eine Bilipidschicht aus Lipidmolekülen mittels Painting, durch Vesikelfusion oder Langmuir-Blodgett/ Langmuir-Schäfer-Technik hergestellt sein. Solche künstlichen Lipidmembranen werden oft als Modelle natürlicher Membranen verwendet. Sie dienen z.B. als Umgebung zur Untersuchung von Membranproteinen, die z.B. zwischen zwei durch die Membran getrennten Kompartimenten Ladungen durch die Membran transportieren. Eine solche Membran kann aber auch eine natürliche, biologische Membran sein, die über der mindestens einen Mikroapertur aufgebracht wird. Dies kann erfolgen, indem ein im wesentlichen planarer Abschnitt ("patch") der Membran einer biologischen Zelle verwendet wird oder indem eine komplette, behandelte oder unbehandelte biologische Zelle verwendet wird, die auf der mindestens einen Mikroapertur aufliegt. Die Dicke der molekularen Schicht hat insbesondere molekulare Abmessungen, kann insbesondere zwischen 1 nm und 100 nm liegen.

Unter einer Mikroapertur wird der offene Querschnitt verstanden, der sich z.B. durch eine Öffnung, z.B. eine Vertiefung oder ein Loch, in einer -insbesondere planaren-Oberfläche der Oberseite des Trägersubstrats ergibt. Die Form des Mikroaperturumrisses ist vorzugsweise kreisförmig, ellipsoid, dreieckförmig, viereckförmig oder mehreckförmig. Der maximale, minimale oder durchschnittliche Durchmesser der einzelnen Mikroapertur ist vorzugsweise kleiner als 1000 µm und liegt vorzugsweise zwischen 500 nm und 500 µm, vorzugsweise zwischen 2 µm und 250 µm, vorzugsweise zwischen 2 µm und 50 µm, vorzugsweise zwischen 2 µm und 10 µm, oder zwischen 5 µm und 150 µm. Bei solchen bevorzugten Mikroaperturgrößen (Mikroaperturen) kann eine Molekülschicht über der Mikroapertur erzeugt werden, was bei makroskopischen Aperturen mit Durchmessern von mehreren Millimetern in der Regel nicht möglich ist.

Solche Mikroaperturen können durch selektives Entfernen einer lichtempfindlichen Schicht, z.B. Fotolack, die auf der Oberseite des Trägers angebracht ist, mittels optischer Litographie erzeugt werden, wie z.B. von dem Dokument "Baaken et al." oder der US 2009/0167288 A1 beschrieben. Die Mikroapertur kann aber auch den Rand eines Lochs bilden, das sich von der Oberseite bis zur Rückseite des Trägersubstrats erstreckt. Dies kann z.B. durch chemisches Ätzen oder durch Bestrahlung mit Laser- oder sonstigen hochenergetischen Strahlen erreicht werden.

Die Anordnung der Anzahl N von Mikroaperturen entspricht vorzugsweise einem Array, vorzugsweise einem periodischen Gitter, in dem sich die Position der Mikroaperturen bzw. der Mikroapertur-Zentren durch einen oder wenige Gitterparameter beschreiben lässt. Die Anordnung in einem periodischen Gitter hat Vorteile beim Entwurf einer parallelisierten Sensorik, in der viele möglichst gleichartige Messstellen geschaffen werden sollen. Die Mikroaperturen können aber auch in einem nicht-periodischen oder nicht vollständig periodischen Muster angeordnet sein.

Das Trägersubstrat ist vorzugsweise aus Glas hergestellt oder weist Glas auf. Es kann aber auch aus einem Halbleitermaterial bestehen oder dieses zumindest aufweisen, z.B. Si/SiO2. Andere Materialien sind ebenfalls möglich. Die Oberseite des Trägersubstrats weist vorzugsweise eine Beschichtung auf. Diese ist vorzugsweise hydrophob, kann aber auch hydrophil sein. Der Vorteil einer hydrophoben Oberseite ist, dass sich viele Arten von Bilipidschichten auf solchen Oberflächen besonders zuverlässig ausbilden.

Unter einer "hydrophoben" Grenzschicht wird im Rahmen der vorliegenden Erfindung eine Schicht verstanden, auf der ein Wassertropfen einen Kontaktwinkel von mindestens 70° aufweist, vorzugsweise mindestens 80°, 85° oder 90°, vorzugsweise zwischen 80° und 130° oder zwischen 90° und 120°. Insofern kann die vorliegende Definition des Begriffs "hydrophob" breiter gewählt sein als allgemein in der Literatur üblich, wo der Begriff meist Kontaktwinkel von größer als 90° bezeichnet. Solche Kontaktwinkel (Innenwinkel des Wassertropfens auf dem Substrat) lassen sich leicht mittels im Handel erhältlicher Kontaktwinkelmessgeräte oder durch Auswertung lichtmikroskopischer Querschnittsbilder der Tropfen ermitteln (Raumtemperatur, Standardbedingungen). Bei einer hydrophilen Grenzschicht sind die Kontaktwinkel jeweils vorzugsweise zwischen 70° und 0°, 80° und 0°, 85° und 0° oder 90° und 0°.

Vorzugsweise weist ein Trägersubstrat mindestens eine Mikrokavität auf, oder vorzugsweise ein Array von Mikrokavitäten auf, wobei eine oder jede Mikrokavität nach oben offen ist und in einer der genannten Mikroaperturen in der Oberseite des Trägersubstrats mündet. Die zu messende molekulare Membran lässt sich dann so bilden, dass sie die mindestens eine Mikroapertur oder mehrere Mikroaperturen überdeckt. Eine Mikrokavität ist eine Vertiefung in der Oberseite, deren Tiefe dieselbe Größe wie ein möglicher genannter Mikroaperturdurchmesser haben kann, oder tiefer oder weniger tief sein kann. Jeder Querschnitt durch die Vertiefung (in einer Ebene parallel zur Ebene der Mikroapertur) weist vorzugsweise denselben Querschnitt auf wie die Mikroapertur, welche die Mikrokavität nach oben öffnet. Die Mikrokavität kann insbesondere zylinderartig oder quaderartig sein. Sie kann aber auch hohlkegel(stumpf)förmig sein oder eine andere Form mit veränderlichem Querschnitt aufweisen.

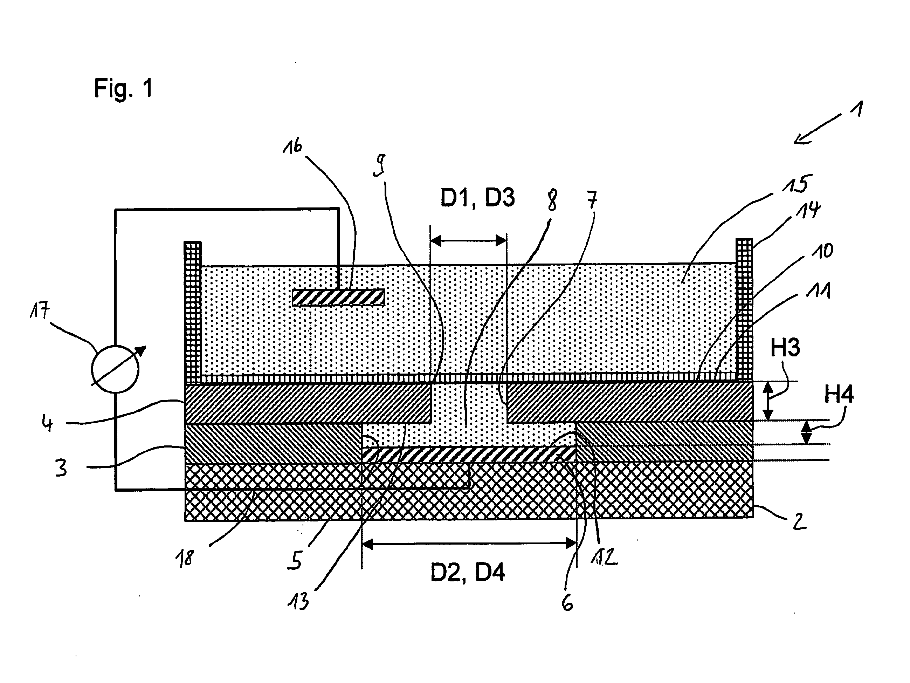

Die Mikrostrukturvorrichtung ist vorzugsweise als Messanordnung ausgebildet oder ist Teil einer Messanordnung. Eine solche Messanordnung weist vorzugsweise die Mikrostrukturvorrichtung auf und mindestens ein erstes Kompartiment auf, nämlich einen Raumbereich oder eine Kammer. Eine Mikrokavität kann als "zweites Kompartiment" einer Messanordnung dienen. Die Mikrostrukturvorrichtung weist ferner vorzugsweise mindestens einen Wandabschnitt auf, der oberhalb des Trägersubstrats angeordnet ist und mit dem Trägersubstrat bzw. dessen Beschichtung(en) die Kammer bzw. das erste Kompartiment definiert, in der ein Flüssigkeitsvolumen von einigen Mikolitern und vorzugsweise bis zu einem Milliliter anordenbar ist. Somit kann die Mikrostrukturvorrichtung einen Kammerabschnitt zur Aufnahme eines solchen Flüssigkeitsvolumens, insbesondere eines ersten und eines zweiten Lösungsmittels (Elektrolyts) aufweisen. Das erste und das zweite Kompartiment sind über die mindestens eine Mikroapertur verbunden. Die Kompartimente sind trennbar, insbesondere elektrisch trennbar, indem oberhalb der Mikroapertur eine Membran angeordnet wird, z.B. eine Bilipidschicht. Diese Membran trennt die elektrolytgefüllten Kompartimente elektrisch dicht voneinander. Es ist vorzugsweise innerhalb des zweiten Kompartiments ein Elektrolyt zum Kontaktieren der Unterseite einer auf der Mikroapertur angeordneten Molekülschicht anordenbar oder angeordnet. In der Mikrokavität ist vorzugsweise die Elektrode angeordnet, mittels der der Elektrolyt im zweiten Kompartiment elektrisch kontaktiert wird (entsprechend der Anordnung in Fig. 1). Ferner ist vorzugsweise mindestens eine Gegenelektrode im ersten Kompartiment angeordnet, wobei in diesem ersten Kompartiment ein Elektrolyt anordenbar ist oder angeordnet ist. Mit dieser Messanordnung, welche die Mikrostrukturvorrichtung aufweist, kann eine Spannungsklemmtechnik-Messanordnung realisiert werden.

Vorzugsweise weist die Messanordnung oder die Mikrostrukturvorrichtung mindestens eine Sensoreinrichtung auf, die insbesondere einen Sensor für elektrophysiologische Untersuchungen an der Molekülschicht, insbesondere Bilipidschicht, aufweist. Die Sensoreinrichtung kann diese Elektrode im zweiten Kompartiment aufweisen, die diesseits der Mikroapertur am Trägersubstrat angeordnet ist, und kann ferner mindestens eine weitere Elektrode (Gegenelektrode) auf der anderen Seite der Mikroapertur aufweisen, die im ersten Kompartiment im Elektrolyt oberhalb der Molekülschicht angeordnet ist. Eine solche Elektrode ist vorzugsweise eine Redoxelektrode, vorzugsweise eine Redoxelektrode "zweiter Art", z.B. eine Ag/AgCI-Elektrode oder eine Kalomel-Elektrode. Die Elektrode ist vorzugsweise eine nicht polarisierbare Elektrode, die einen einfachen Übergang der ionischen Ladungsträger im Elektrolyten in elektronische Ladungsträger im Metall erlaubt. Vorzugsweise werden dazu Ag/AgCI-Elektroden, vorzugsweise in Kombination mit chlorionenhaltigen Messlösungen (Elektrolyt) verwendet.

Diese Sensoreinrichtung ist vorzugsweise zur Durchführung der Spannungsklemmtechnik ausgebildet, mittels der bei konstant gehaltener Spannung kleinste Ströme im Nanoamperebereich und darunter gemessen werden können, insbesondere im Picoamperebereich, z.B. unter Verwendung eines Voltage-Clamp-Verstärkers oder eines Patch-Clamp-Verstärkers (z.B ein Axopatch 200B, Axon Instruments, Foster City, CA, betrieben im "resistive feedback mode"). Die Sensoreinrichtung kann ein Array von Sensoren aufweisen, die im Trägersubstrat oder an dessen Oberfläche angeordnet sein können.

Als Lösungsmittel, nämlich als Elektrolyt, der unterhalb und/oder oberhalb der Membran (Molekülschicht) platziert wird, kommen insbesondere Salzlösungen in Frage, insbesondere physiologische Salzlösungen, welche die elektrophysiologische Vermessung der Molkülschicht, z.B. Lipid-Membran, und darin enthaltener, ladungstransportierender Poren, z.B. Kanalproteine, erlauben. Geeignete Lösungsmittel, insbesondere zur Durchführung von Messungen mittels Spannungsklemmtechnik, ergeben sich z.B. aus dem Dokument "Baaken et al." oder der US 2009/0167288 A1.

Geeignete amphiphile Moleküle zur Herstellung der Molekülschichten sind insbesondere Lipide, insbesondere zur Bildung von membranartigen Doppellipidschichten geeignete Lipide, wie sie z.B. u.a. in dem Dokument "Baaken et al." oder in US 2009/0167288 A1 genannt werden.

Bevorzugte Ausgestaltungen der erfindungsgemäßen Vorrichtung können nachfolgend auch der Beschreibung des erfindungsgemäßen Verfahrens entnommen werden und umgekehrt.

Das erfindungsgemäße Verfahren zur Herstellung einer erfindungsgemäßen Mikrostrukturvorrichtung weist zumindest die Schritte auf, insbesondere ohne die Reihenfolge der Schritte dabei zwingend festzulegen: - Bereitstellen eines Trägersubstrats; - Aufbringen mindestens einer Elektrode auf das Trägersubstrat; - Aufbringen einer (strukturierbaren) Schicht oberhalb des Trägersubstrats; - Ausbilden der mindestens einen Mikrokavität durch selektives Entfernen von Abschnitten dieser (strukturierbaren) Schicht, wobei diese Abschnitte die Mikrokavität wenigstens zum Teil oder vollständig bilden.

Vorzugsweise weist das Verfahren den Schritt auf, dass diese (strukturierbare) Schicht direkt auf dem Trägersubstrat oder einer Schicht des Trägersubstrats gebildet wird. Alternativ weist das Verfahren den Schritt auf, dass diese strukturierbare Schicht indirekt auf dem Trägersubstrat gebildet wird, indem diese strukturierbare Schicht zunächst auf einem zweiten, separaten Trägersubstrat gebildet wird und danach von diesem zweiten Trägersubstrat auf das erste Trägersubstrat oder eine Schicht auf dem zweiten Trägersubstrat übertragen wird (vorzugsweise mittels "Bonding", wie erläutert im Dokument "Baaken et al.").

Weitere Eigenschaften und Merkmale der Mikrostrukturvorrichtung und zur Herstellung der Mikrostrukturvorrichtung, Materialien, Methoden, Messanordnungen und Beispiele zur Messung an Membranen können dem Dokument "Baaken et al." oder der US 2009/0167288 A1 entnommen werden

Weitere bevorzugte Ausgestaltungen des erfindungsgemäßen Verfahrens und der erfindungsgemäßen Mikrostrukturvorrichtung ergeben sich aus der nachfolgenden Beschreibung der Ausführungsbeispiele in Zusammenhang mit den Figuren. Gleiche Bezugszeichen bezeichnen im wesentlichen gleiche Bauteile.
Fig. 1 zeigt schematisch ein erstes Ausführungsbeispiel der erfindungsgemäßen Mikrostrukturvorrichtung zur Messung an einer Membran in einem schematischen, senkrechten Querschnitt.
Fig. 2a zeigt ein zweites Ausführungsbeispiel der erfindungsgemäßen Mikrostrukturvorrichtung als schematischen, senkrechten Querschnitt.
Fig. 2b zeigt die Mikrostrukturvorrichtung der Fig. 2a als schematische Aufsicht.
Fig. 3a zeigt ein drittes Ausführungsbeispiel der erfindungsgemäßen Mikrostrukturvorrichtung als schematischen, senkrechten Querschnitt.
Fig. 3b zeigt die Mikrostrukturvorrichtung der Fig. 3a als schematische Aufsicht.
Fig. 4 zeigt eine mit einer erfindungsgemäßen Mikrostrukturvorrichtung gemessene Spannungskurve V(t).
Fig. 5 zeigt eine mit einer erfindungsgemäßen Mikrostrukturvorrichtung gemessene Stromkurve I(t).
Fig. 6 zeigt schematisch zwei Ausführungsbeispiele des erfindungsgemäßen Verfahrens zur Herstellung der erfindungsgemäßen Mikrostrukturvorrichtung.

Figur 1 zeigt die Mikrostrukturvorrichtung 1, die als Messanordnung zur elektrophysiologischen Messung an einer Membran 11, nämlich einer Bilipidmembran, mittels Spannungs-Klemmtechnik ausgebildet ist. Die Mikrostrukturvorrichtung 1 weist ein Trägersubstrat 2 aus Glas auf, auf dem eine erste Schicht 3 aus Fotolack (SU8) durch Aufschleudern (Spin-Coating) aufgebracht wurde. Die erste Schicht 3 weist eine hohlzylinderförmige Aussparung 5 auf, die insbesondere durch eine fotolithographische Methode erzeugt wurde innerhalb der auf der Oberseite des Trägersubstrats 2 eine Redoxelektrode 6 angebracht ist. Oberhalb der ersten Schicht 3 ist eine zweite Schicht 4 aus Fotolack (SU8) aufgebracht, die durch Aufschleudern (Spin-Coating) des flüssigen Fotolacks auf der ersten Schicht 3 hergestellt wurde. Der Verfahrensschritt, die zweite Schicht aus Epoxidharz auf einer ersten Schicht aus Epoxidharz durch Aufschleudern zu erzeugen, wobei die erste Schicht insbesondere bereits mikrostrukturiert ist, nämlich insbesondere bereits die Aussparungen 5 aufweist, hat sich als überraschend erfolgreich herausgestellt. Die zweite Schicht wird dabei fest, das heißt nicht zerstörungsfrei trennbar, mit der ersten Schicht verbunden und bildet eine planare Oberseite 10 der Mikrostrukturvorrichtung bzw. des durch die Schichten 3, 4 erweiterten Trägersubstrats 2, welches die Membran 11 trägt. Die zweite Schicht 4 weist eine hohlzylinderförmige Aussparung 7 auf, die mit der Aussparung 5 innerhalb der Verbundschicht aus erster Schicht 3 und zweiter Schicht 4 ein Innenvolumen bildet. Das Innenvolumen der Mikrokavität 8 wird durch den durch die Aussparung 5 definierten ersten Raumabschnitt durch den durch die Aussparung 7 definierten zweiten Raumabschnitt gebildet. Die Innenwände der Aussparungen 5 und 7 in den Schichten 3 und 4, ein Abschnitt 13 der Unterseite der zweiten Schicht und die als Boden dienende Kontaktseite 12 der Elektrode 6 definieren die Mikrokavität 8 und dienen als erstes Kompartiment 8 beziehungsweise als Kammer 8 für einen flüssigen Elektrolyt.

Die nach oben offene Mikrokavität 8 mündet an ihrer Oberseite in die Mikroapertur 9, die in der planaren Oberseite 10 der Mikrostrukturvorrichtung angeordnet ist und die eine kreisförmige Fläche der Mikroapertur 9 mit dem Durchmesser D1 aufweist. D1 ist der charakteristische Durchmesser der Mikroapertur 9.

Die Elektrode 6 hat die Form eines flachen Zylinders und ist formschlüssig im Bereich der Aussparung 5 in der Mikrokavität 8 angeordnet. Ihre Kontaktseite 12 grenzt an das Innenvolumen der Mikrokavität 8 an, um das elektrische Kontaktieren eines in der Mikrokavität angeordneten Elektrolyten zur ermöglichen. Die Kontaktseite 12 der Elektrode 6 weist einen Durchmesser D2 auf. D2 ist der charakteristische Durchmesser der Kontaktseite 12 der Elektrode 6. D2 ist etwa dreimal so groß wie D1. Verwendet man die Mikrostrukturvorrichtung 1 zur Messung an einer molekularen Membran 11 mittels Spannungsklemmtechnik, ergibt es sich, dass der durch Elektrode und Membran fließende Strom an der Elektrode 6 eine relativ geringe Stromdichte bewirkt, da deren Kontaktseite 12 mit dem Elektrolyt einen relativ großen charakteristischen Durchmesser D2 aufweist. Die relativ geringe elektrische Stromdichte (Strom/Fläche) der Kontaktseite 12 bringt den Vorteil mit sich, dass die pro Fläche elektrochemisch induzierten Reaktionen an der Kontaktseite der Elektrode reduziert werden, welche die Elektrode 6 mit der Zeit umbilden und so deren Eigenschaften ändern. Dadurch weist die Elektrode konstantere elektrische Eigenschaften auf, insbesondere eine geringere Drift und eine verlängerte Messdauer.

Der durch die erste Aussparung 5 und die Kontaktseite 12 definierte erste Raumabschnitt ist hohlzylinderförmig und weist einen Durchmesser D4 auf. D4 ist der charakteristische Durchmesser des ersten Raumabschnitts der Mikrokavität 8. Der durch die zweite Aussparung 7 definierte zweite Raumabschnitt ist auch hohlzylinderförmig und weist einen Durchmesser D3 auf. D3 ist der charakteristische Durchmesser des ersten Raumabschnitts der Mikrokavität 8. Aufgrund der zur Bildung der Mikrokavität 8 gewählten Geometrie ist D1=D3 und D2=D4. Der erste Raumabschnitt weist eine Höhe H4 auf. H4 ist die charakteristische Höhe des ersten Raumabschnitts der Mikrokavität 8. Der zweite Raumabschnitt weist eine Höhe H3 auf. H3 ist die charakteristische Höhe des zweiten Raumabschnitts der Mikrokavität 8. Aufgrund der zur Bildung der Mikrokavität 8 gewählten Geometrie ist H4<H3. Insbesondere haben sich solche relativ kleinen Werte für H4 bewährt, um die Elektrode 6 zuverlässig mit dem Elektrolyt in der Mikrokavität 8 zu kontaktieren.

Oberhalb der Oberseite 10 des Trägersubstrats 2 bilden Wandabschnitte 14 das zweite Kompartiment 15, das eine Kammer zur flüssigkeitsdichten Aufnahme eines Elektrolyten bildet. Das erste Kompartiment 8 und das zweite Kompartiment 15 sind im Bereich der Mikroapertur 9 elektrisch wird durch die Membran 11 getrennt, die dicht auf der Mikroapertur 9 aufliegt. Mittels einer Gegenelektrode 16, die im Elektrolyt des zweiten Kompartiments 15 angeordnet ist, kann nun unter weiterer Verwendung von Mess-Elektronik 17 eine Spannung-Klemmtechnik-Messanordnung realisiert werden. Die Mikrokavität 8 wird am Boden nach unten durch die innere Redoxelektrode 6 (z.B. Ag/AgCl) abgeschlossen. Diese ist über eine z.B. lithographisch erzeugte Zuleitung 18 elektrisch mit der Sensorelektronik 17 der Sensoreinrichtung verbunden. Im Elektrolyt der Kammer 15 befindet sich die Redoxelektrode 16, die als Referenzelektrode dient. Durch Spannungsklemmtechnik, bei der eine elektrische Spannung über die Membran z.B. mithilfe eines Patch-Clamp-Verstärkers konstant gehalten wird, können geringste Stromänderungen eines Stroms von Ladungen "q" durch die Membran detektiert werden, welche als BLM die Mikroapertur 9 überspannt. Diese Ladungen können auf dem Ionentransport durch undichte Stellen der Zellmembran beruhen oder auf Poren, z.B. Kanalproteine in der Zellmembran zurückgehen.

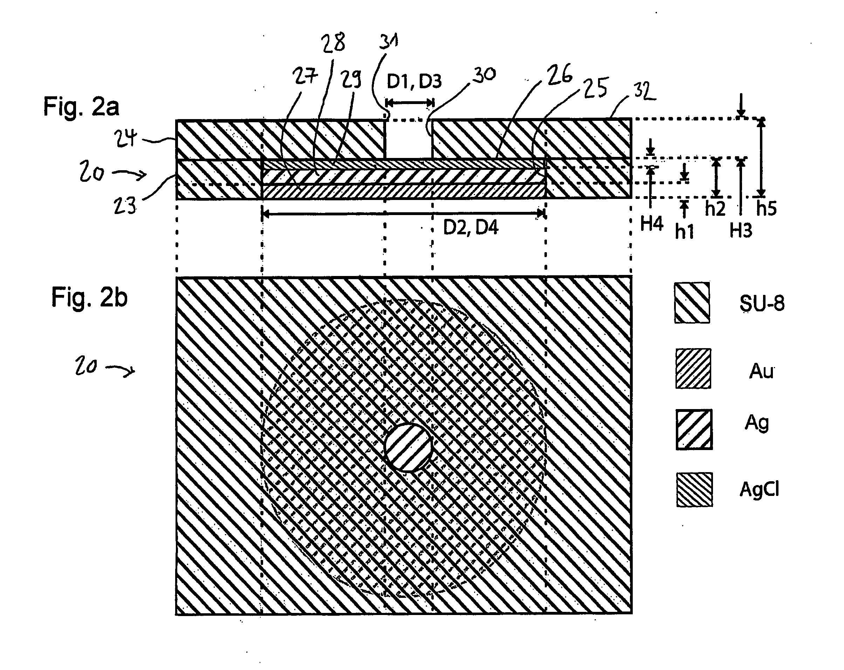

Figur 2a zeigt ein zweites Ausführungsbeispiel die Mikrostrukturvorrichtung 20. Die Mikrostrukturvorrichtung 20 weist ein Trägersubstrat (nicht gezeigt) auf, auf dem die erste Schicht 23 aus SU8-Fotolack durch Aufschleudern auf das Trägersubstrat aufgebracht wurde. Die erste Schicht 23 weist eine hohlzylinderförmige Aussparung 25 auf, die fotolithographisch erzeugt wurde. Innerhalb der Aussparung 25 ist die Elektrode 26 eingepasst. Die Elektrode 26 besteht aus mehreren Schichten. Sie weist eine erste Schicht 27 aus Gold auf, die fotolithographisch erzeugt wurde und die das Glasträgersubstrat (nicht gezeigt) kontaktiert und mit diesem verbunden ist. Diese Schicht dient als Starterschicht zum Aufbringen einer Schicht 28 aus Silber, z.B. durch Aufdampfen. Ausgehend von der Schicht 28 aus Silber wird durch elektrochemische Abscheidung von Silberchlorid aus einer Silbernitratlösung die Schicht 29 aus Silberchlorid auf der Schicht 28 aus Silber erzeugt. Auf diese Weise erhält man in der Aussparung 25 eine Silber/Silberchlorid-Elektrode 26, die als Redoxelektrode zum elektrischen Kontaktieren eines chloridionenhaltigen Elektrolyten dient.
Oberhalb der ersten Schicht 23 wird die zweite Schicht 24 aus Fotolack SU8 aufgeschleudert. In der zweiten Schicht 24 ist die Aussparung 30 angeordnet, die in die Mikroapertur 31 in der Oberseite 32 des durch die Schichten 23, 24 erweiterten Trägersubstrats mündet, welche dem Tragen der Membran dient.

Das Innenvolumen der Mikrokavität der Mikrostrukturvorrichtung 20 wird durch den ersten Raumabschnitt, der durch die erste Aussparung 25 in der ersten Schicht 23 definiert ist, und dem zweiten Raumabschnitt, der durch die zweite Aussparung 30 in der zweiten Schicht 24 definiert ist, gebildet. Dies ist insofern überraschend, als die erste Aussparung 25 im wesentlichen vollständig von der Elektrode 26 ausgefüllt wird. Es hat sich aber überraschend gezeigt, dass der zusammenhängende Hohlraum bzw. die zusammenhängenden Hohlräume zwischen den Poren der nanoporösen Silberchloridschicht 29, die mit dem zweiten Raumabschnitt 30 verbunden sind, einen zum elektrischen Kontaktieren der Elektrode 26 durch einen flüssigen Elektrolyt geeigneten ersten Raumabschnitt festlegen. Diesem ersten Raumabschnitt kann ein charakteristischer Durchmesser D4 und eine charakteristische Höhe H4 zugeordnet werden, die von den entsprechenden realen Maßen der Silberchloridschicht 29 abweichen, nämlich kleiner sind. Die Silberchloridschicht 29 weist eine Zusammensetzung aus Körnern mit einer Korngrößen von typischerweise 50 nm bis 500 nm auf. Das Innenvolumen von deren Zwischenräumen bildet das Volumen dieses ersten Raumabschnitts. Je nach Polarität der Elektrode 26 im Betrieb der Mikrostrukturvorrichtung kann sich die Dicke der Silberchloridschicht 29 insbesondere in einem silberchloridhaltigen Elektrolyt ändern, so dass sich das Volumen des ersten Raumabschnitts vergrößern und verkleinern kann. Es ist H4<H3.

Typische Dimensionen der Mikrostrukturvorrichtung 20 können z.B. wie folgt gewählt werden: Charakteristischer Durchmesser D1 der Mikroapertur = Durchmesser der Mikroapertur und charakteristischer Durchmesser D3 des zweiten Raumabschnitts = Durchmesser des zweiten Raumabschnitts: D1 = D3 = 3 µm - 20 µm; Charakteristischer Durchmesser D2 der Elektrode = Durchmesser der Elektrode und charakteristischer Durchmesser D4 des ersten Raumabschnitts = Durchmesser des ersten Raumabschnitts: D2 = D4 = 10 µm - 100 µm, z.B. 60 µm; Gesamthöhe h5 der Verbundschicht aus erster Schicht 23 und zweiter Schicht 24: h5 = 14 - 20 µm; Gesamthöhe h2 der Elektrode 26: h2 = 7 µm - 10 µm; Höhe h1 der Schicht 27 der Elektrode: ca. 0,2 µm; Höhe der Schicht 28 der Elektrode: 6 µm - 9 µm; Höhe der Silberchloridschicht 29: variierend, 10 nm bis 2 µm.

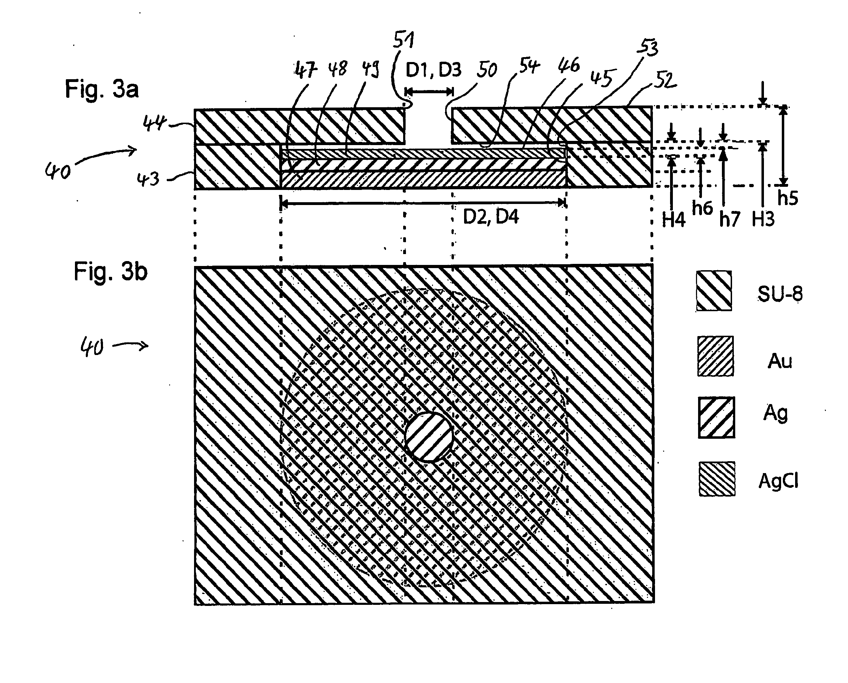

Fig. 3a zeigt als weiteres Ausführungsbeispiel die Mikrostrukturvorrichtung 40, die ähnlich der Mikrostrukturvorrichtung 20 ist und analog hergestellt wurde. Die Mikrostrukturvorrichtung 40 weist ein Trägersubstrat (nicht gezeigt) auf, auf dem die erste Schicht 43 aus SU8-Fotolack aufgebracht wurde. Die erste Schicht 43 weist eine hohlzylinderförmige Aussparung 45 auf. Innerhalb der Aussparung 45 ist die Elektrode 46 eingepasst. Die Elektrode 46 besteht aus mehreren Schichten. Sie weist eine erste Schicht 47 aus Gold auf und die das Glasträgersubstrat (nicht gezeigt) kontaktiert und mit diesem verbunden ist. Diese Schicht dient als Starterschicht zum Aufbringen einer Schicht 48 aus Silber. Ausgehend von der Schicht 48 aus Silber wird die Schicht 49 aus Silberchlorid auf der Schicht 48 aus Silber erzeugt. Auf diese Weise erhält man in der Aussparung 45 eine Silber/Silberchlorid-Elektrode 46, die als Redoxelektrode zum elektrischen Kontaktieren eines chloridionenhaltigen Elektrolyten dient.

Oberhalb der ersten Schicht 43 wird die zweite Schicht 44 aus Fotolack SU8 aufgeschteudert. In der zweiten Schicht 44 ist die Aussparung 50 angeordnet, die in die Mikroapertur 51 in der Oberseite 52 des durch die Schichten 43, 44 erweiterten Trägersubstrats mündet, welche dem Tragen der Membran dient.

Das Innenvolumen der Mikrokavität der Mikrostrukturvorrichtung 40 wird durch den ersten Raumabschnitt, der durch die erste Aussparung 45 in der ersten Schicht 43 definiert ist, und dem zweiten Raumabschnitt, der durch die zweite Aussparung 50 in der zweiten Schicht 44 definiert ist, gebildet. Der zweite Raumabschnitt wird im Gegensatz zur Mikrostrukturvorrichtung 20 nicht nur durch die Nanoporosität der Silberchloridschicht 49 erzeugt, der eine charakteristische Höhe h6 zugeordnet werden kann, sondern auch durch den freien, hohlzylinderartigen Teilabschnitt 53 der Aussparung 45, der zwischen der dem Innenvolumen der Mikrokavität zugewandten Kontaktseite der Elektrode 46 und Abschnitten 54 der Unterseite der zweiten Schicht 44 gebildet wird. Dieser Teilabschnitt weist eine Höhe h7 auf. Der zweite Raumabschnitt weist also eine charakteristische Höhe H4 auf, die der realen Höhe h7 des freien Teilabschnitts 53 zuzüglich der charakteristischen Höhe h6 der Silberchloridschicht 49 entspricht. Durch den freien Teilabschnitt 53 ist die Kontaktseite der Elektrode 46 für den Elektrolyt noch besser zugänglich. Es ist H4< H3.

Bei beiden Arten der Mikrostrukturvorrichtung 20 und 40 konnte die Stabilität der Elektroden konnte durch die relativ große Elektrodenfläche (D2>D1) und, insbesondere dadurch bedingt, das Vorhalten von einer im Vergleich zu kleineren Elektroden deutlich höheren Materialmenge an nanoporösem AgCl deutlich verbessert werden.

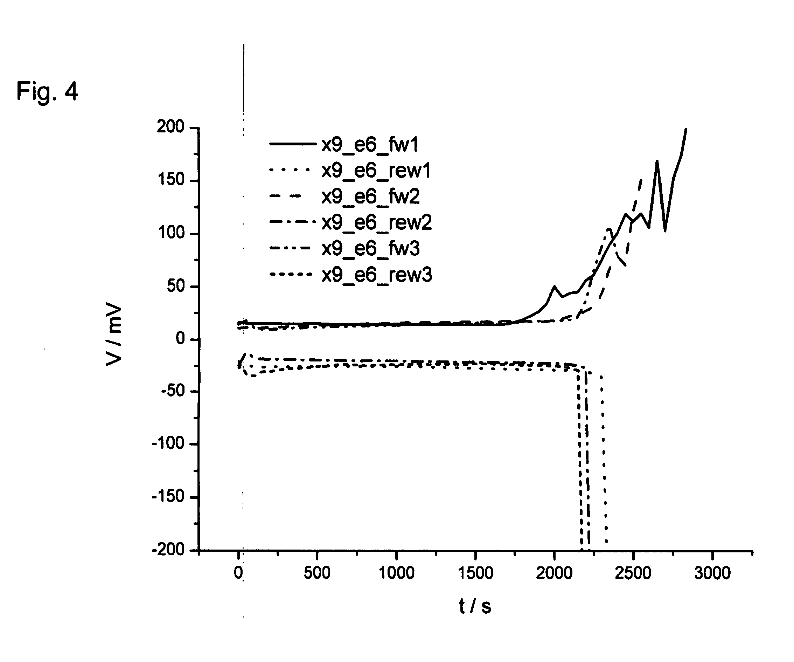

Fig. 4 zeigt eine mit einer Mikrostrukturvorrichtung 20 oder 40 (mit Ag/AgCl Elektroden (D2=50µm)) gemessene Spannungskurve V(t). Dies ist ein Stabilitätstests der Ag/AgCl Elektroden, eingebettet in eine Isolatorschicht (SU8). Die Graphen beschreiben den Verlauf der für einen konstanten Strom von +-10 nA benötigten Spannung über die Zeit.

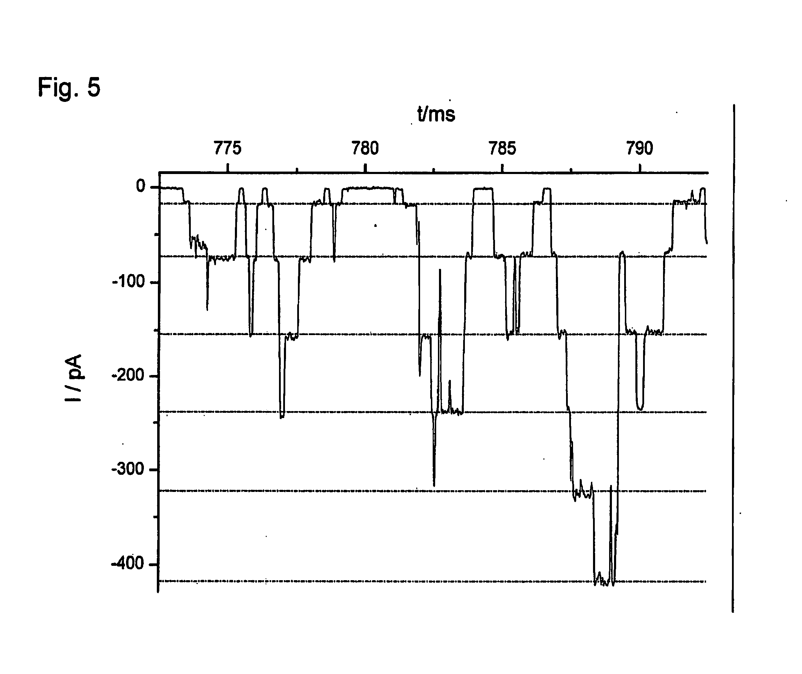

Die Elektrode wurde über 3 Zyklen als Kathode bzw. als Anode geschaltet und jeweils bis zum Stabilitätsverlust betrieben. Mit den so erhaltenen Elektroden konnten bereits erste Messungen mit sehr hoher Qualität an Bilipidschichten durchgeführt werden, die Alamethicin (siehe Fig. 5) oder Botulinustoxin C2 enthielten. Hier zeigte sich, dass die erwartete Verbesserung der elektrischen Parameter voll verwirklicht wurde.

Es ist in Fig. 4 die für ein Einprägen eines Stromes von +/-10 nA erforderliche Spannung über die Zeit dargestellt. Die Elektrode wurde zyklisch umgeschaltet, zwischen Polung als Anode und als Kathode. Im einen Fall wächst auf der Oberfläche eine Silberchloridschicht auf, was nach einer gewissen Zeit, aufgrund des hohen elektrischen Widerstandes zu einem deutlichen Anstieg der für einen Strom von 10 nA erforderlichen Spannung führt. An diesem Punkt wurde der Messaufbau umgepolt, d.h. die Mikroelektrode als Kathode geschaltet. Es wird jetzt an der Oberfläche Silberchlorid zu Silber reduziert. Ist kein Silberchlorid mehr vorhanden steigt der Widerstand und die Spannung wieder stark an. Die Elektrode ist nicht mehr stabil und wurde wieder umgepolt usw. Insgesamt wurden 3 Zyklen (hin und zurück) gefahren.

Es ist zu erkennen, dass die Elektroden, unerheblich, ob als Kathode oder Anode geschaltet, über mehr als eine halbe Stunde den Strom ohne eine erhebliche Abweichung in der dazu erforderlichen Spannung halten können. Damit konnte gezeigt werden, dass die Stabilitätskriterien für die Messungen an Zellen bzw. an hochleitfähigen bakteriellen Poren mehr als erfüllt sind. Verwendet man einen Strom von 1 nA, so verlängert sich z.B. die Zeit, die für die Messungen in eine Polarisationsrichtung zu Verfügung steht, auf z.B. ca. 6 Stunden. Bemerkenswerterweise können die Elektroden nach dem Stabilitätsverlust in eine Polarisationsrichtung bei Betrieb mit der jeweils entgegengesetzten Triebkraft wieder vollständig hergestellt werden, wie der Durchlauf mehrerer Zyklen in Fig. 4 verdeutlicht.

Fig. 5 zeigt eine mit einer Mikrostrukturvorrichtung 20 oder 40 gemessene Stromkurve I(t). Es handelt sich um typische Stromverläufe beim Spannungsklemmen von Alamethicin-vermittelten Strömen in einer Lipiddoppelschicht. Bemerkenswert sind die außergewöhnlich hohen Sealwiderstände (mehrere 100 GOhm), die auf den SU8 Chips der Mikrostrukturvorrichtungen 20, 40 erhalten wurden. Dadurch wird das Signal-zu-Rausch-Verhältnis nochmals deutlich verbessert.

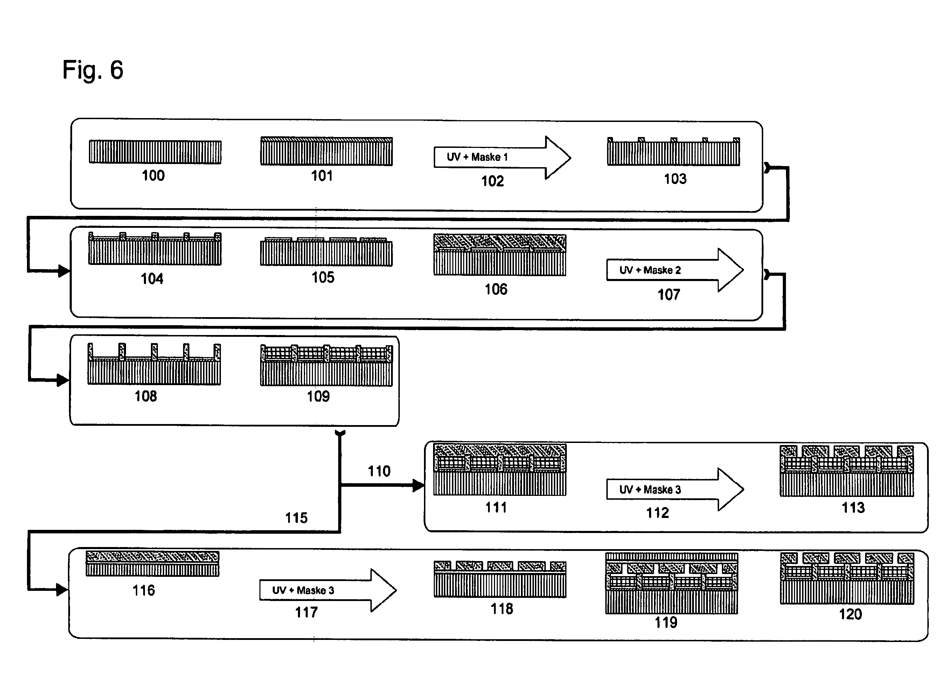

Fig. 6 zeigt schematisch zwei Ausführungsbeispiele des erfindungsgemäßen Verfahrens zur Herstellung der erfindungsgemäßen Mikrostrukturvorrichtung, insbesondere zur Herstellung einer solchen vom Typ der Mikrostrukturvorrichtung 20 oder 40, jeweils als Mehrfach-Elektrode in Array-Anordnung. Weitere Details zur Herstellung der Mikrostrukturvorrichtung können z.B. dem Dokument "Baaken et al." entnommen werden.

In einem ersten Schritt 100 wird das planare Trägersubstrat bereitgestellt, zum Beispiel ein transparentes Substrat aus, zum Beispiel, Glas. Im Schritt 101 wird eine Schicht aus Fotolack SU8 (Umkehrlack) durch Spin-Coating aufgeschleudert. Im Schritt 102 wird auf dieses beschichtete Trägersubstrat eine "Maske 1" gelegt und mit UV Licht belichtet. Im Schritt 103 wird die belichtete Schicht entwickelt und auf diese Weise Abschnitte der Schicht selektiv entfernt. Auf diese Mikrostrukturschicht wird im Schritt 104 eine haftungsvermittelnde beziehungsweise galvanische Startschicht aus Chrom, Gold aufgedampft. Im Schritt 105 wird der Fotolack, der das Trägersubstrat beim metallischen Bedampfen selektiv abgeschirmt hatte, abgelöst (Strippen, Lift-Off), so dass eine metallische Mikrostruktur zurückbleibt. Diese wird im Schritt 106 durch Aufschleudern von Fotolack erneut beschichtet. Dies ist die erste Schicht des Trägersubstrats, in der die ersten Aussparungen 25, 45 (Fig. 2a und 3a) ausgebildet werden, in denen die Elektroden 26, 46 eingebracht werden. Dies erfolgt, indem im Schritt 107 eine "Maske 2" auf die Fotolackbeschichtung gelegt wird und UV- belichtet wird. Im Schritt 108 wird die Fotolackbeschichtung über den Chrom-Gold-Bereichen durch Entwickeln des belichteten Fotolacks selektiv entfernt. Im Schritt 109 werden die Chrom-Gold-Bereiche mit Silber beschichtet, so dass die einzelnen Elektroden 26, 46 der Mehrfach-ElektrodenAnordnung ihre Silberschicht 28, 48 erhalten.

Mit SU8 lassen sich durch Aufschleudern Schichtdicken z.B. von 1 - 200 µm erreichen, die durch die Viskosität des Fotolacks und die Drehgeschwindigkeiten leicht einzustellen sind. Für die erste Schicht kann man bei z.B. D2=60 µm der Elektroden insbesondere auf bis zu 200 µm hochgehen (abhängig davon, wieviel Silber man vorhalten will). Bei der zweiten Schicht sind z.B. 5-10 µm sinnvoll, um auch kleine Aperturen beim Entwickeln noch vollständig öffnen zu können.

Zum Aufbringen der zweiten Schicht 24, 44 aus Fotolack, die letztlich die Mikroaperturen 31, 51 definiert, bestehen zwei alternative Verfahrensausgestaltungen 110, 115:

Als sehr effektiv hat sich die Schrittfolge 110 herausgestellt. Dabei wird im Schritt 111 die zweite Schicht aus Fotolack (vorzugsweise auch SU8) direkt auf die erste Schicht aus Fotolack (vorzugsweise auch SU8) aufgeschleudert, welche die Elektrodenmikrostruktur bereits aufweist. Überraschenderweise zeigte sich in Versuchen, dass es möglich ist, durch das einfache Aufschleudern des Polymers (z.B. Epoxidharz, z.B. SU8) auf die erste Schicht aus demselben Polymer-Material eine zweite Schicht aufzubringen, die sich mit der ersten Schicht nach Vernetzung stabil verbindet. Die Materialien der beiden Schichten können aber auch unterschiedlich sein, solange sich eine ausreichend stabile Verbindung der beiden Schichten ergibt. Auf die zweite Schicht wird im Schritt 112 die "Maske 3" aufgelegt und UV-belichtet. Im Schritt 113 wird der belichtete Fotolack durch Entwickeln selektiv entfernt, so dass sich die Aussparungen 30, 50 in der zweiten Schicht bilden, welche die zweiten Raumabschnitte der Mikrokavitäten darstellen. Damit ist eine Mikrostrukturvorrichtung geschaffen, die eine Anzahl von N Mikrokavitäten aufweist (z.B. N=4 in der Figur oder auch z.B. N=16), wobei jede Mikrokavität eine Elektrode aufweist.

Alternativ zur Verfahrensabfolge 110 kann die Abfolge 115 von Verfahrensschritten gewählt werden, um die zweite Schicht mit Mikroaperturen auf der ersten Schicht aufzubringen. Dazu wird auf einem separaten Substrat, zum Beispiel einem Glasträger, eine entfernbare Opferschicht aufgebracht und darauf eine zweite Fotolackschicht (z.B. SU8) aufgeschleudert. Im Schritt 117 wird auf diese Fotolackschicht die "Maske 3" aufgelegt und UV belichtet. Der belichtete Fotolack wird im Schritt 118 entwickelt, um die Bereiche für die Aussparungen 30, 50 (Mikroaperturen) selektiv zu entfernen. Im Schritt 119 wird die zweite Schicht mit der ersten Schicht aus vorzugsweise demselben Fotolack in einem speziellen Verbindungsprozess fest verbunden. Dieser Verbindungsprozess wird als "thermisches Bonding" bezeichnet. Zwei (beschichtete) Substrate werden dabei unter hohem Druck und hoher Temperatur zusammengefügt. Im vorliegenden Fall 150 °C bis 200 °C und 2000 bis 4000 mBar Druck. Im Schritt 120 wird der Opferschicht aufgelöst und auf diese Weise das zweite Substrat abgelöst. Damit ist auf alternativem Weg 115 eine Mikrostrukturvorrichtung geschaffen, die eine Anzahl von N Mikrokavitäten aufweist (z.B. N=4 in der Figur oder auch z.B. N=16), wobei jede Mikrokavität eine Elektrode aufweist.

Durch die vorliegende Erfindung kann eine zuverlässige und stabile Plattform für die Hochdurchsatzelektrophysiologie bereitgestellt und kostengünstig auf dem Markt angeboten werden. Die Möglichkeit einer hohen Integrationsdichte und die möglichen, hervorragenden Signal-Rauschabstände bieten Anwendern gegenüber bekannten Vorrichtungen erhebliche Vorteile. Der Endnutzer wird in die Lage versetzt, schnell und mit hohem Durchsatz Membranströme aller Art zu messen. Solche Messungen spielen eine wachsende Rolle in der pharmazeutischen Wirkstofffindung sowie der Biotechnologie.

## Patentansprüche

1. Mikrostrukturvorrichtung (1) zur Messung an molekularen Membranen (11), aufweisend
ein Trägersubstrat (2) mit einer Oberseite (10) zum Tragen der Membran,
mindestens eine Mikrokavität (8) des Trägersubstrats zum Aufnehmen eines Elektrolyten, wobei die Mikrokavität nach oben offen ist und in einer Mikroapertur (9) in der Oberseite des Trägersubstrats mündet,
wobei die Mikroapertur einen ersten charakteristischen Durchmesser D1 aufweist,
mindestens eine Elektrode (6), die zumindest teilweise in der Mikrokavität angeordnet ist und die eine an das Innenvolumen der Mikrokavität angrenzende Kontaktseite (12) zum Kontaktieren eines Elektrolyten aufweist,
wobei diese Kontaktseite (12) der Elektrode einen charakteristischen Durchmesser D2 aufweist, der größer ist als D1,
wobei die Mikrokavität einen oberen Raumabschnitt aufweist, der nach oben von der Mikroaperturfläche begrenzt ist und der im Querschnitt einen charakteristischen Durchmesser D3 aufweist und dass die Mikrokavität einen unteren Raumabschnitt aufweist, der unterhalb des oberen Raumabschnitts liegt und der in diesem Querschnitt einen charakteristischen Durchmesser D4 aufweist, wobei D4>D3 und wobei der betrachtete Querschnitt durch den geometrischen Mittelpunkt einer planaren Mikroaperturfläche führt und zu dieser senkrecht liegt,
**dadurch gekennzeichnet, dass**
der obere Raumabschnitt eine charakteristische Höhe H3 aufweist und der untere Raumabschnitt eine charakteristische Höhe H4 aufweist, wobei H4 kleiner ist als H3.

2. Mikrostrukturvorrichtung nach Anspruch 1, **dadurch gekennzeichnet, dass** D2 größer ist als D1*1,5 (D1 multipliziert mit einem Faktor c=1,5).

3. Mikrostrukturvorrichtung nach Anspruch 1 oder 2, **dadurch gekennzeichnet, dass** das Trägersubstrat eine erste Schicht (3) aufweist, die im wesentlichen unterhalb der Mikroapertur angeordnet ist und innerhalb der die Elektrode angeordnet ist.

4. Mikrostrukturvorrichtung nach Anspruch 3, **dadurch gekennzeichnet, dass** das Trägersubstrat eine zweite Schicht (4) aufweist, die im wesentlichen unterhalb der Mikroapertur angeordnet ist und innerhalb der die Mikrokavität wenigstens teilweise oder im wesentlichen vollständig angeordnet ist.

5. Mikrostrukturvorrichtung nach Anspruch 4, **dadurch gekennzeichnet, dass** die Mikrokavität zumindest teilweise auch in der ersten Schicht angeordnet ist.

6. Mikrostrukturvorrichtung nach mindestens einem der vorherigen Ansprüche, **dadurch gekennzeichnet, dass** die Kontaktseite zumindest in einem Abschnitt oder vollständig eine mikrostrukturierte oder nanostrukturierte Oberfläche oder eine mikroporöse oder nanoporöse Oberfläche aufweist.

7. Verfahren zur Herstellung einer Mikrostrukturvorrichtung gemäß einem der Ansprüche 1 bis 6, umfassend die Schritte, ohne Festlegung der Reihenfolge:
Bereitstellen eines Trägersubstrats;
Aufbringen mindestens einer Elektrode auf das Trägersubstrat;
Aufbringen einer Schicht oberhalb des Trägersubstrats;
Ausbilden der mindestens einen Mikrokavität durch selektives Entfernen von Abschnitten dieser Schicht, wobei diese Abschnitte die Mikrokavität wenigstens zum Teil oder vollständig bilden.

8. Verfahren gemäß Anspruch 7, wobei diese Schicht direkt auf dem Trägersubstrat gebildet wird oder, alternativ, indirekt auf dem Trägersubstrat gebildet wird, indem diese Schicht zunächst auf einem zweiten Trägersubstrat gebildet wird und danach von diesem zweiten Trägersubstrat auf das Trägersubstrat übertragen wird.

## Claims

1. A microstructure device (1) for measuring molecular membranes (11) comprising
a carrier substrate (2) having an upper surface (10) for supporting the membrane,
at least one microcavity (8) of the carrier substrate for receiving an electrolyte, wherein the microcavity is upwardly open and opens out into a microaperture (9) in the upper surface of the carrier substrate,
wherein the microaperture comprises a first characteristic diameter D1,
at least one electrode (6) which is at least partially arranged within the microcavity and which comprises a contact side (12), that is adjoining the interior volume of the microcavity, for contacting an electrolyte,
wherein this contact side (12) of the electrode comprises a characteristic diameter D2 which is greater than D1,
wherein the microcavity comprises an upper space portion which is upwardly bounded by the microaperture surface and which has in the cross-section a characteristic diameter D3 and in that the microcavity comprises a lower space portion which is located below the upper space portion and which has in this cross-section a characteristic diameter D4, wherein D4>D3 and wherein the contemplated cross-section runs through the geometrical center of a planar microaperture surface and is perpendicular thereto,
**characterized in that**
the upper space portion comprises a characteristic height H3 and the lower space portion comprises a characteristic height H4, wherein H4 is less than H3.

2. A microstructure device according to claim 1, **characterized in that** D2 is greater than D1*1.5 (D1 multiplied by a factor c=1.5).

3. A microstructure device according claim 1 or 2, **characterized in that** the carrier substrate comprises a first layer (3) which is essentially located under the microaperture and within which the electrode is arranged.

4. A microstructure device according to claim 3, **characterized in that** the carrier substrate comprises a second layer (4) which is essentially located under the microaperture and within which the microcavity is arranged at least partially or essentially completely.

5. A microstructure device according to claim 4, **characterized in that** the microcavity is at least partially also arranged within the first layer.

6. A microstructure device according to at least one of the preceding claims, **characterized in that** the contact side comprises at least in a section or completely a microstructured or nanostructured surface or a microporous or nanoporous surface.

7. A method for producing a microstructure device according to any one of the claims 1 to 6 comprising the steps, without determining the order:
Providing a carrier substrate;
Applying at least one electrode onto the carrier substrate;
Applying a layer above said carrier substrate;
Forming the at least one microcavity by selectively removing portions of this layer, wherein these portions form the microcavity at least partially or completely.

8. A method according to claim 7, wherein this layer is directly formed on the carrier substrate or alternatively is indirectly formed on the carrier substrate by at first forming this layer on a second carrier substrate and thereafter transferring it from said second carrier substrate onto the carrier substrate.

## Revendications

1. Dispositif à microstructure (1) destiné à effectuer des mesures sur des membranes moléculaires (11), comprenant
un substrat de support (2) avec un côté supérieur (10) pour supporter la membrane,
au moins une microcavité (8) du substrat de support destinée à accueillir un électrolyte, la microcavité étant ouverte vers le haut et débouchant dans un micro-orifice (9) dans le côté supérieur du substrat de support,
le micro-orifice présentant un premier diamètre caractéristique D1,
au moins une électrode (6), qui est agencée tout au moins partiellement dans la microcavité et présente une face de contact (12) adjacente au volume intérieur de la microcavité en vue d'entrer en contact avec un électrolyte,
cette face de contact (12) de l'électrode présentant un diamètre caractéristique D2, qui est supérieur à D1,
la microcavité présentant un secteur spatial supérieur, qui est délimité vers le haut par la surface du micro-orifice et qui, en section, présente un diamètre caractéristique D3, et la microcavité présentant un secteur spatial inférieur, qui se situe sous le secteur spatial supérieur et qui présente, dans cette section, un diamètre caractéristique D4, avec D4>D3, et la section considérée passant par le centre géométrique d'une surface de micro-orifice plane et étant perpendiculaire à celle-ci,
**caractérisé en ce que**
le secteur spatial supérieur présente une hauteur caractéristique H3 et le secteur spatial inférieur une hauteur caractéristique H4, H4 étant inférieur à H3.

2. Dispositif à microstructure selon la revendication 1, **caractérisé en ce que** D2 est supérieur à D1*1,5 (D1 multiplié par un facteur c = 1,5).

3. Dispositif à microstructure selon la revendication 1 ou la revendication 2, **caractérisé en ce que** substrat de support présente une première couche (3), qui est essentiellement agencée sous le micro-orifice et à l'intérieur de laquelle est agencée l'électrode.

4. Dispositif à microstructure selon la revendication 3, **caractérisé en ce que** le substrat de support présente une deuxième couche (4), qui est essentiellement agencée sous le micro-orifice et à l'intérieur de laquelle est agencée, au moins en partie ou sensiblement en totalité, la microcavité.

5. Dispositif à microstructure selon la revendication 4, **caractérisé en ce que** la microcavité est au moins en partie également agencée dans ladite première couche.

6. Dispositif à microstructure selon l'une au moins des revendications précédentes, **caractérisé en ce que** la face de contact présente, au moins dans un secteur ou en totalité, une surface micro-structurée ou nano-structurée ou une surface microporeuse ou nano-poreuse.

7. Procédé de fabrication d'un dispositif à microstructure selon l'une des revendications 1 à 6, comprenant les étapes suivantes, sans ordre fixe défini :
préparation et mise à disposition d'un substrat de support ;
application d' au moins une électrode sur le substrat de support ;
application d'une couche sur le dessus du substrat de support ;
formation de ladite au moins une microcavité par enlèvement sélectif de secteurs de cette couche, ces secteurs formant la microcavité au moins en partie ou en totalité.

8. Procédé selon la revendication 7, d'après lequel cette couche est formée directement sur le substrat de support ou, en variante, indirectement sur le substrat de support par le fait que cette couche est tout d'abord formée sur un deuxième substrat de support et est ensuite transférée, à partir de ce deuxième substrat de support, sur le substrat de support.
